(19) Europäisches Patentamt
European Patent Office
Office européen des brevets

(11) Publication number : **0 244 810 B1**

(12) # EUROPEAN PATENT SPECIFICATION

(45) Date of publication of patent specification : **11.03.92 Bulletin 92/11**

(51) Int. Cl.$^5$ : **C07D 263/22**

(21) Application number : **87106458.0**

(22) Date of filing : **05.05.87**

(54) **Process for producing 2-oxazolidinones.**

The file contains technical information submitted after the application was filed and not included in this specification

(30) Priority : **06.05.86 JP 101935/86**

(43) Date of publication of application : **11.11.87 Bulletin 87/46**

(45) Publication of the grant of the patent : **11.03.92 Bulletin 92/11**

(84) Designated Contracting States : **BE CH DE FR GB IT LI NL**

(56) References cited : **DE-C- 883 902** **DE-C- 917 972**

(73) Proprietor : **MITSUI TOATSU CHEMICALS, Inc.**
**2-5 Kasumigaseki 3-chome**
**Chiyoda-Ku Tokyo 100 (JP)**

(72) Inventor : **Kajimoto, Nobuyuki**
**Mitsui Toatsu, Hirabaru Apartment 432-GO,**
**300 Hirabaru-Cho, Omuta-shi, Fukuoka (JP)**
Inventor : **Nagata, Tereuyuki**
**154 Shiragane-cho,**
**Omuta-shi, Fukuoka (JP)**
Inventor : **Wada, Masaru**
**1807-158 Oaza Kanugi,**
**Omuta-shi, Fukuoka (JP)**
Inventor : **Tamaki, Akihiro**
**53-6 Sasahara-cho 2-chome,**
**Omuta-shi, Fukuoka (JP)**

(74) Representative : **Gervasi, Gemma, Dr. et al**
**NOTARBARTOLO & GERVASI Srl 33, Viale Bianca Maria**
**I-20122 Milano (IT)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

BACKGROUND OF THE INVENTION

1. Field of the Invention

This invention relates to an improvement in the process for producing 2-oxazolidinones by reacting amino-alcohols and/or their hydrochlorides with phosgene.

2-Oxazolidinones are useful as an intermediate for high molecular materials, pharmaceuticals, pesticides, etc. or as an addition component to non-protonic polar solvents, various compositions, etc.

2. Description of the Related Art

As to the process for producing 2-oxazolidinones, various processes have so far been proposed, and the processes of reacting amino-alcohols with phosgene have also already been known.

For example, according to a process of carrying out phosgenation reaction in chloroform solvent, the objective product is obtained with a yield of 31.6 % (Ber. der Deut. Chem. G., vol. 51, page 1662; German Patent 917972). Further, according to a process of reacting amino-alcohols with phosgene-pyridine complex in an ether, the yield of the objective product is 60% (Chem. Ber. Vol. 89, page 2565). Still further, according to a process of carrying out phosgenation reaction in dioxane solvent, the objective product is obtained with yields of 20% and 35% (Makromol. Chem. Vol. 94, pages 132-142 and Israel J. of Chem., vol. 6, No. 2, pages 147-150).

As described above, the above-mentioned processes of obtaining 2-oxazolidinones by reacting amino-alcohols with phosgene except for the process of using phosgene-pyridine complex are very low in yield; hence the processes have been entirely unsatisfactory as commercial processes.

Further, as to the process of using phosgene-pyridine complex, the complex must be expressly prepared in advance so that the operability is troublesome and the yield is still unsatisfactory.

The present inventors have made extensive research on a commercial production process of 2-oxazolidinones using phosgene and as a result have found the following facts.

Usually the reaction of using phosgene has been carried out to the utmost under water-free condition, and it has been known that when a dehydrochlorinating agent is present in an aqueous solution, phosgene is easily hydrolysed. Thus it has been expected that a large excess of phosgene might be required for the reaction thereof with amino-alcohols. Surprisingly enough, however, even when water and a dehydrochlorinating agent are present in the reaction of amino-alcohols with phosgene, the quantity of phosgene used in about 1.1 to 1.5 times its chemical equivalent is sufficient, and also the objective 2-oxazolidinones are obtained with a commercially satisfactory yield.

Further, at that time, when the reaction is carried out while thy pH of the reaction system is kept within a definite range with a dehydrochlorinating agent in the presence of water, the objective compound is obtained with a further higher yield.

SUMMARY OF THE INVENTION

The object of the present invention is to provide a process for producing 2-oxazolidinones by reacting amino-alcohols with phosgene with a commercially high yield and also in a simplified manner. The present invention resides in a process for producing a 2-oxazolidinone expressed by the formula (II)

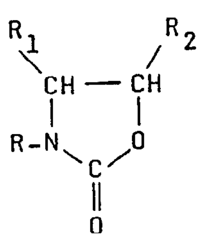

(II)

wherein R represents hydrogen atom or a lower alkyl group and $R_1$ and $R_2$ are the same as or different from one another and each represent hydrogen atom or a lower alkyl group, by reacting an amino-alcohol expressed by the formula (I)

$$R\text{-}NHCHR_1CHR_2OH \qquad (I)$$

wherein R, $R_1$ and $R_2$ are each as defined above, and/or its hydrochloride, with phosgene, which process comprises carrying out said reaction substantially in the presence of water and a dehydrochlorinating agent.

DESCRIPTION OF PREFERRED EMBODIMENTS

According to the present invention, since the reaction is carried out substantially in the presence of water, that is, in water medium, the amino-alcohol hydrochloride successively formed through hydrochloric acid byproduced during the phosgenation reaction is not deposited out of the reaction system at the time of the reaction, but it is dissolved in water; hence in any case it is possible to carry out the reaction in a uniform state.

Thus it is possibile to very easily control the pH of the reaction liquid at the time of the reaction.

Further since a dehydrochlorinating agent is simultaneously used at the time of the reaction, the dehydrochlorinating agent not only functions effectively for catching the byproduced hydrochloric acid, but also when the reaction is carried out while thhe pH is kept within a range of 3.0 to 10.0 by adding the dehydrochlorinating agent, the objective product is obtained in a water solvent wherein hydrolysis of phosgene is expected, with an unexpectably high yield. The reason is that it is possible to inhibit formation of N,N′-di(hydroxyalkyl) ureas as an intermolecular byproduct which are expected to be formed within a higher pH range and formation of chlorocarbonic acid esters as a byproduct which are expected to be resultantly formed within a lower pH range, by controlling the pH.

In the above formula (I), R represents hydrogen atom or a lower alkyl group. Examples of the lower alkyl group are methyl, ethyl, propyl, butyl, etc. Further, $R_1$ and $R_2$ each represent hydrogen atom or a lower alkyl group.

Examples of the lower alkyl group are methyl, ethyl, etc..

Examples of the amino-alcohols expressed by the formula (I) are 2-aminoethanol, 2-(methylamino)ethanol, 2-(ethylamino) ethanol, 2-[(1-methylethyl)amino]ethanol, 2-(butylamino)ethanol, 3-amino-2-butanol, 1-amino-2-butanol, 1-ethylamino-2-propanol, etc. These amino-alcohols may be obtained from the corresponding olefin oxides and ammonia or the corresponding alkylamines. These amino-alcohols may be directly phosgenated or first converted into their hydrochlorides, followed by reacting these with phosgene.

However, according to the process wherein the reaction is carried out while the pH is kept within a range of 3.0 to 10.0, it is advantageous to feed amino-alcohols partly together with their hydrochlorides from the beginning of the reaction. Preferably amino-alcohols are in advance dissolved in water and the solution is adjusted to a desired pH with hydrochloric acid.

According to the present invention, the reaction is carried out in the presence of a substantial quantity of water. Thus water is placed in the reactor in advance or dropwise introduced into the rearctor together with a dehydrochlorinating agent, for example, in the form of an aqueous solution of an alkali metal compound.

The quantity of water used has no particular limitation, but a quantity enough to keep a uniform reaction is preferred; thus 0.5 to 50 times, preferably 3 to 30 times the weight of amino-alcohols are preferred. As the dehydrochlorinating agents used in the present invention, alkali metal compounds such as sodium hydroxide, potassium hydroxide, sodium carbonate, etc. or tertiary amines such as trimeth,ylamine, triethylamine, N,N-dimethylaniline, N,N-diethylaniline, pyridine, picoline, etc.. are preferred.

If no dehydrochlorinating agent is used, the raw material amino-alcohols themselves become an agent for catching byproduced hydrochloric acid so that the phosgenation reaction rate of the N atom of the raw material becomes very low.

Further, the reaction temperature in the present invention has no particular limitation, but it is preferred to be in the range of 0° to to 70°C.

In a preferred embodiment of the present invention wherein the reaction is carried out while the pH of the reaction system is kept within a definite range, the reaction is carried out while the pH at the time of the reaction is kept within a range of 3.0 to 10.0, preferably 4.0 to 9.0, more preferably 5.0 to 8.0. If the pH is higher, the quantity of phosgene itself consumed by the dehydrochlorinating agent is remarkable. Moreover, while the phosgenation reaction rate of phosgene with the N atom of amino-alcohols becomes greater, an intermolecular urea byproduct (N,N′-(dihydroxyalkyl)urea) is formed so that the selectivity relative to amino-alcohols becomes lower.

To the contrary if the pH is lower, the quantity of phosgene itself consumed by the dehydrochlorinating agent is small, but the proportion in which the N atom of the raw material constitutes the hydrochloride increases so that the phosgenation reaction rate of phosgene relative to the N atom decreases. Further, since the alcohol residual group of the amino-alcohols is converted into the corresponding chloroformate through the phosgenation, the selectivity relative to the amino-alcohols is resultantly reduced.

The quantity of phosgene used in the process of the present invention is preferred to be usually an equivalent ratio of 1.1 to 1.5 based on the raw material amino-alcohols.

Examples of the 2-oxazolidinones of the formula (II) obtained according to the process of the present invention are 2-oxazolidinone, 3-methyl-2-oxazolidinone, 3-ethyl-2-oxazolidinone, 3-(1-methylethyl)-2-oxazolidinone, 3-butyl-2-oxazolidinone, 4,5-dimethyl-2-oxazolidinone, 5-ethyl-2-oxazolidinone, 3-ethyl-5-methyl-2-oxazolidinone, etc. A usual preferred embodiment of the process of the present invention is as follows:

water and the amino-alcohols are introduced into a reactor equipped with a reflux condenser, a thermometer, a phosgene-blowing tube, a dropping funnel, an electrode for pH measurement and a stirrer. The reaction may be started as it is, but preferably hydrochloric acid is added to adjust the pH of the liquid feed to about 3 to 10. While the resulting liquid is agitated at a suitable temperature, phosgene is introduced through the phosgene-blowing tube and at the same time a solution of a dehydrochlorinating agent is dropwise added through the dropping funnel. The pH of the reaction liquid is thereby kept in the range of 3.0 to 10.0, preferably 4.0 to 9.0, more preferably 5.0 to 8.0.

After completion of the blowing and dropwise addition, unreacted phosgene, if necessary, is purged by nitrogen gas and the resulting material is subjected to a conventional process such as extraction and/or distillation and the objective product is taken out.

The process of the present invention exhibits the following superior effectiveness as compared with conventional processes of obtaining 2-oxazolidinones by reacting amino-alcohols with phosgene:

(1) the objective compounds are obtained with a higher yield than those of conventional processes; hence the process has a great commercial value in terms of a production process of low cost.

(2) the cheapest water is used as solvent and as to the quantity of phosgene used in the reaction, an equivalent ratio of 1.1 to 1.5 relative to the raw material amino-alcohols is sufficient; hence the process is economical.

(3) the reaction mixture is uniform, control during the reaction is easy and the post-treatment of the reaction such as filtering operation, etc. are unnecessary; hence the process is superior in operability.

The present invention will be described in more detail by way of Examples and Comparative examples.

## EXAMPLE 1

Into a 2 l flask equipped with a reflux condenser, a thermometer, a dropping funnel, a phosgene-blowing tube, an electrode for pH measurement and a stirrer were introduced water (500 ml), 2-(methylamino)ethanol (75.1 g, 1.00 mol) and 36% HCl aqueous solution (101.3 g, 1.00 mol), and on the other hand, 30% NaOH aqueous solution was introduced into the dropping funnel. While the reaction liquid was kept at 25°C, phosgene was blown in the flask through the phosgene-blowing tube with stirring for 1.5 hour at a rate of 100 g/hr. At the same time, 30% NaOH aqueous solution was dropwise added through the dropping funnel so as to keep the pH of the reaction mixture at 7.0-8.0. The quantity of 30% NaOH aqueous solution dropped was 653 g (4.90 mols).

Samples were taken from the resulting reaction mass and subjected to gas chromatography to analyze the raw material 2-(methylamino)ethanol and the product 3-methyl-2-oxazolidinone. As a result, unreacted 2-(methylamino)ethanol (6.9 g) and 3-methyl-2-oxazolidinone (73.6 g) were present in the reaction mass and the conversion was 90.8 %, the selectivity, 93.1 % and the yield, 84.5%.

To this reaction mass was added 49% NaOH aqueous solution to make the pH 13 or more, followed by twice subjecting it ot extraction with iso-propylalcohol (100 g/once), separating the resulting oil layer and distilling it to obtain 3-methyl-2-oxazolidinone (69.9 g).

## EXAMPLE 2

Into a 200 ml flask equipped with a reflux condenser, a thermometer, a dropping funnel a phosgene-blowing tube, an electrode for pH measurement and a stirrer were introduced water (50 ml), aminoethanol (6.11 g, 0.100 mol) and 30% HCl aqueous solution (10.1 g, 0.100 ml), and on the other hand, 30% NaOH aqueous solution was introduced into the dropping funnel. While the reaction liquid was kept at 25°C, phosgene was blown in the flask through the phosgene-blowing tube with stirring for 1.5 hour at a rate of 10 g/hr. At the same time. 30% NaOH aqueous solution was dropwise added through the dropping funnel so as to keep the pH of the reaction mixture at 7.0-8.0. The quantity of 30% NaOH aqueous solution dropped was 64 g (0.48 mol).

Samples were taken from the resulting reaction mass and subjected to gas chromatography to quantitatively analyze the raw material aminoethanol and the product 2-oxazolidinone. As a result, the conversion was 96.1%, the selectivity, 95.2% and the yield, 91.5%.

EXAMPLES 3-16

Using the same apparatus as in Example 2 and varying the raw material amino-alcohol, the quantity of phosgene blown and the reaction pH, reactions were carried out, followed by subjecting the raw material amino-alcohols and products 2-oxazolidinones to quantitative analysis according to gas chromatography as in Example 2 and calculating the conversions, selectivities and yields.

The results are shown in Table 1 together with those of Examples 1 and 2.

Comparative example 1

Into a 200 ml flask equipped with a reflux condenser, a thermometer, a phosgene-blowing tube and a stirrer were introduced dioxane (50 ml) and 2-(methylamino)ethanol (7.51 g, 0.100 mol). While the reaction liquid was kept at 25°C, phosgene was blown in the flask through the phosgene-blowing tube with stirring for 1.5 hour at a rate of 10 g/hr.

Samples were taken from the resulting reaction mass, followed by subjecting the raw material 2--(methylamino)ethanol and the product 3-methyl-2-oxazolidinone to quantitative analysis. As a result, the conversion was 99.1 %, the selectivity, 19.7 % and the yield, 18.9%.

Comparative example 2

Into a 200 ml flask equipped with a reflux condenser, a thermometer, a phosgene-blowing tube and a stirrer were introduced water (50 ml) and 2-(methylamino)ethanol (7.51 g, 0.100 mol). While the reaction liquid was kept at 25°C, phosgene was blown in the flask through the phosgene-blowing tube with stirring from 1.5 hour at a rate of 10 g/hr.

Samples were taken from the resulting reaction mass, followed by subjecting the raw material 2--(methylamino)ethanol and the product 3-methyl-2-oxazolidinone to quantitative analysis. As a result, the conversion was 99.8%, the selectivity, 20.7% and the yield, 20.7%.

Table 1

| Example | Feed | | | | | | Blowing | | | Reaction temperature | Dehydrochlorinating agent | | | Reaction PH | Reaction product | | | |
|---|---|---|---|---|---|---|---|---|---|---|---|---|---|---|---|---|---|---|
| | Amino-alcohols | | | Water | 36%HCl | | Blowing rate | Total blown quantity | | | Kind | Total dropped quantity | | | 2-Oxazolidinones | Conversion | Selectivity | Yield |
| | Name of raw materials | Wt.(g) | Mol | ml | Wt.(g) | HCl mol | g/hr | Wt.(g) | Mol | (°C) | | Wt.(g) | Base mol | | | (%) | (%) | (%) |
| 1 | 2-(Methylamino)-ethanol | 75.1 | 1.00 | 500 | 101.3 | 1.00 | 100 | 150 | 1.5 | 25 | 30% NaOH | 653 | 4.90 | 7-8 | 3-Methyl-2-oxazolidinone | 90.8 | 93.1 | 84.5 |
| 2 | Aminoethanol | 6.11 | 0.100 | 50 | 10.1 | 0.100 | 10 | 15 | 0.15 | " | " | 64 | 0.48 | " | 2-Oxa-zolidinone | 96.1 | 95.2 | 91.5 |
| 3 | 2-(Ethylamino)-ethanol | 8.92 | " | " | " | " | " | " | " | " | " | 63 | 0.47 | " | 3-Ethyl-2-oxa-zolidinone | 97.0 | 92.8 | 90.0 |
| 4 | 2-[(1-Methyl-ethyl)amino]-ethanol | 10.32 | " | " | " | " | " | " | " | " | " | 65 | 0.49 | " | 3-(1-Methyl-ethyl)-2-oxa-zolidinone | 88.1 | 97.5 | 85.9 |
| 5 | 2-(Butylamino)-ethanol | 11.72 | " | " | " | " | " | " | " | " | " | 67 | 0.50 | " | 3-Butyl-2-oxa-zolidinone | 92.5 | 94.3 | 87.2 |
| 6 | 3-Amino-2-butanol | 8.92 | " | " | " | " | " | " | " | " | " | 65 | 0.48 | " | 4,5-Dimethyl-2-oxa-zolidinone | 94.9 | 95.7 | 90.8 |
| 7 | 1-Amino-2-butanol | 8.92 | " | " | " | " | " | " | " | " | " | 64 | 0.48 | " | 5-Ethyl-2-oxa-zolidinone | 92.3 | 93.3 | 86.1 |
| 8 | 1-Ethylamino-2-propanol | 10.32 | " | " | " | " | " | " | " | " | " | 64 | 0.48 | " | 3-Ethyl-5-methyl-2-oxa-zolidinone | 93.6 | 96.0 | 89.9 |

Table 1 (continued)

| Example | Feed: Amino-alcohols: Name of raw materials | Feed: Amino-alcohols: Wt.(g) | Feed: Amino-alcohols: Mol | Feed: Water: ml | Feed: 36%HCl: Wt.(g) | Feed: 36%HCl: HCl mol | Blowing: Blowing rate: g/hr | Blowing: Total blown quantity: Wt.(g) | Blowing: Total blown quantity: Mol | Reaction temperature (°C) | Dehydrochlorinating agent: Kind | Dehydrochlorinating agent: Total dropped quantity: Wt.(g) | Dehydrochlorinating agent: Total dropped quantity: Base mol | Reaction PH | Reaction product: 2-Oxazolidinones | Reaction product: Conversion (%) | Reaction product: Selectivity (%) | Reaction product: Theoretical yield (%) |
|---|---|---|---|---|---|---|---|---|---|---|---|---|---|---|---|---|---|---|
| 9 | 2-(Methylamino)-ethanol | 7.51 | 0.100 | 50 | 10.1 | 0.100 | 10 | 15 | 0.15 | 25 | 30% NaOH | 59 | 0.44 | 5-6 | 3-Methyl-2-oxazolidinone | 72.3 | 97.3 | 70.3 |
| 10 | " | " | " | " | " | " | " | " | " | " | " | 53 | 0.40 | 4 | " | 83.1 | 98.1 | 61.9 |
| 11 | " | " | " | " | " | " | " | " | " | " | " | 69 | 0.52 | 9 | " | 95.4 | 73.7 | 70.2 |
| 12 | " | " | " | " | " | " | " | " | " | " | 10% $Na_2CO_3$ | 276 | 0.26 | 7-8 | " | 85.3 | 94.8 | 80.9 |
| 13 | " | " | " | " | " | " | " | " | " | " | 30% KOH | 88 | 0.47 | " | " | 97.1 | 93.7 | 91.0 |
| 14 | " | " | " | " | " | " | " | " | " | " | N,N-dimethylaniline | 60.7 | 0.50 | " | " | 83.9 | 95.1 | 79.8 |
| 15 | " | " | " | " | " | " | " | " | " | " | Pyridine | 38.9 | 0.49 | " | " | 87.4 | 96.2 | 84.1 |
| 16 | " | " | " | " | " | " | " | " | " | " | 30% NaOH | 63 | 0.47 | " | " | 85.6 | 94.5 | 80.9 |

## Claims

1. A process for producing a 2-oxazolidinone expressed by the formula (II)

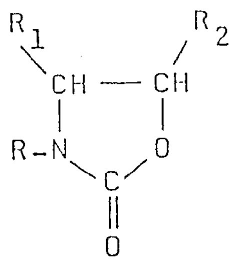

(II)

wherein R represents hydrogen atom or a lower alkyl group and $R_1$ and $R_2$ are the same as or different from one another and each represent hydrogen atom or a lower alkyl group, by reacting an amino-alcohol expressed by the formula (I)

$R\text{-}NHCHR_1CHR_2OH$ (I)

wherein R, $R_1$ and $R_2$ are each as defined above, and/or its hydrochloride, with phosgene, which process comprises carrying out said reaction substantially in the presence of water while keeping the pH of the reaction liquid within a range of 3.0 to 10.0 with a dehydrochlorinating agent in water solvent.

2. A process according to claim 1 wherein said pH is within a range of 5.0 to 8.0.

3. A process according to claim 1 wherein said dehydrochlorinating agent is an alkali metal compound.

4. A process according to claim 1 wherein said dehydrochlorinating agent is a tertiary amine.

## Patentansprüche

1. Verfahren zur Herstellung des 2-Oxazolydon der Formel (II)

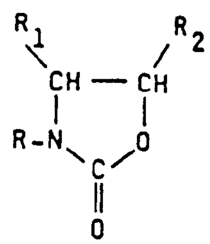

(II)

in welcher R Wasserstoff oder eine niedere Alkylgruppe darstellt und $R_1$ und $R_2$, gleich oder verschieden sind und jedes Wasserstoff oder eine niedere Alkylgruppe darstellt, durch Reaktion eines Amino-alkohols der Formel (I)

$R\text{-}NHCHR_1CHR_2OH$ (I)

in welcher R,$R_1$ und $R_2$ wie oben definiert sind, mit Phosgen, in dem die Reaktion substantiell in Anwesenheit von Wasser durchgeführt wird, während das pH der Reaktionsflüssigkeit zwischen 3,0 und 10,0 durch einem Dehydrochlorierung-Agent in der wässrigen Lösung eingehaltet wird.

2. Verfahren nach Anspruch 1 in welchem das pH zwischen 5,0 und 8,0 ist.

3. Verfahren nach Anspruch 1 in welchem als Dehydrochlorierung-Agent eine Alkalimetall Verbindung verwendet wird.

4. Verfahren nach Anspruch 1 in welchem als Dehydrochlorierung-Agent eine tertiäre Amine verwendet wird.

## Revendications

1. Un procédé de production d'une 2-oxazolidinone représentée par la formule (II) :

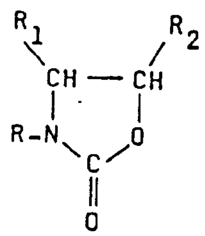

(II)

dans laquelle R représente un atome d'hydrogène ou un groupe alkyle inférieur et $R_1$ et $R_2$ peuvent être identiques ou différents l'un de l'autre et représentent chacun un atome d'hydrogène ou un groupe alkyle inférieur, par réaction d'un aminoalcool répondant à la formule (I) :

$R\text{-}NHCHR_1CHR_2OH$ (I)

dans laquelle R, $R_1$ et $R_2$ sont comme défini précédemment, et/ou son chlorhydrate, avec le phosgène, ledit procédé consistant à conduire ladite réaction substantiellement en présence de l'eau en maintenant le pH du liquide réactionnel dans l'intervalle de 3,0 à 10,0, à l'aide d'un agent de déchlorhydratation dans l'eau comme solvant.

2. Un procédé selon la revendication 1, selon lequel ledit pH est compris dans l'intervalle de 5,0 a 8,0.

3. Un procédé selon la revendication 1, selon lequel ledit agent de déchlorhydratation est un composé de métal alcalin.

4. Un procédé selon la revendication 1, selon lequel ledit agent de déchlorhydratation est une amine tertiaire.